(19) Europäisches Patentamt
European Patent Office
Office européen des brevets

(11) Publication number: **0 051 328**
**B1**

(12) # EUROPEAN PATENT SPECIFICATION

(45) Date of publication of patent specification: **01.08.84**

(51) Int. Cl.³: **C 07 C 29/04**, C 07 C 31/10

(21) Application number: **81201148.4**

(22) Date of filing: **15.10.81**

(54) **Process for the preparation of alcohols.**

(30) Priority: **05.11.80 US 204677**

(43) Date of publication of application:
**12.05.82 Bulletin 82/19**

(45) Publication of the grant of the patent:
**01.08.84 Bulletin 84/31**

(84) Designated Contracting States:
**BE DE FR GB IT NL**

(56) References cited:
FR - A - 2 135 729
GB - A - 389 133
GB - A - 409 676
GB - A - 1 500 667
GB - A - 1 518 461
US - A - 2 472 618

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Slaugh, Lynn Henry**
**610 Winter Oaks**
**Houston Texas 77079 (US)**
Inventor: **Willis, Carl Lesley**
**15922 Red Willow**
**Houston Texas 77084 (US)**

(74) Representative: **Puister, Antonius Tonnis, Mr. et al,**
**P.O. Box 302**
**NL-2501 CH The Hague (NL)**

**EP 0 051 328 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of alcohols by reacting an olefinically unsaturated hydrocarbon with water in the presence of a catalyst.

The hydration of olefins using as catalysts sulphuric and sulphonic acids is known (see, for example, British patent specification 1,518,461). These conventional catalysts have the disadvantage that considerable amounts of by-products such as ketones are produced.

It has now been found that the formation of such by-products is minimized by the use, as catalyst, of an alpha-hydroxysulphonic acid, prepared by reacting a carbonyl compound with sulphur dioxide and water.

Accordingly, the present invention provides a process for the preparation of alcohols, which comprises reacting an olefinically unsaturated hydrocarbon with water in the presence of an alpha-hydroxysulphonic acid prepared by reacting a carbonyl compound of the general formula $R_1R_2CO$, wherein $R_1$ and $R_2$ are individually hydrogen or a substituted or unsubstituted hydrocarbyl group containing up to 7 carbon atoms, with sulphur dioxide and water.

The reaction by which the alpha-hydroxysulphonic acids used as catalysts in the present process are prepared may be represented by the following general equation:

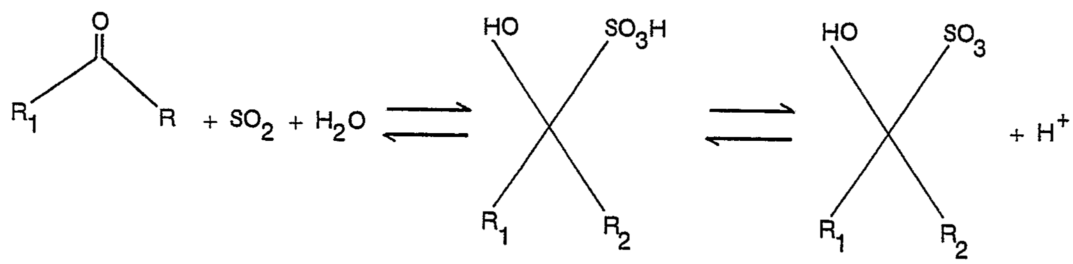
(1)

Illustrative examples of carbonyl compounds useful to prepare the alpha-hydroxysulphonic acids used in this invention are found, where:

$R_1 = R_2 = H$ (formaldehyde)
$R_1 = H$, $R_2 = CH_3$ (acetaldehyde)
$R_1 = H$, $R_2 = CH(CH_3)_2$ (*i*-butyraldehyde)
$R_1 = H$, $R_2 = CCHCHCHO$ (furfural)
$R_1 = H$, $R_2 = C(CH)_4C(OH)$ (salicylaldehyde)
$R_1 = H$, $R_2 = C(CH)_4CH$ (benzaldehyde)
$R_1 = R_2 = CH_3$ (acetone)
$R_1 = CH_3$, $R_2 = CH_2CH_3$ (methyl ethyl ketone)
$R_1 = CH_3$, $R_2 = CHC(CH_3)_2$ (mesityl oxide)
$R_1 = CH_3$, $R_2 = CH_2CH(CH_3)_2$ (methyl *i*-butyl ketone)
$R_1$, $R_2 = (CH_2)_5$ (cyclohexanone)
or $R_1 = CH_3$, $R_2 = CH_2Cl$ (chloroacetone)

A unique feature of these alpha-hydroxysulphonic acids is the easy reversibility of the acid formation according to equation (1), i.e. when heated sulphur dioxide is liberated and the solution becomes neutral. Decreasing the sulphur dioxide pressure for this system induces the same effect. This reversibility provides for the method utilized to remove unutilized acid from the reaction streams. By increasing the temperature or lowering the pressure, the sulphur dioxide can be driven off leaving the carbonyl compound and water. These latter materials can then be removed by conventional means, such as distillation.

Various olefinically unsaturated hydrocarbons are hydrated by the present invention, including linear, branched or cyclic hydrocarbons and including alpha or internal olefins. Suitable compounds are $C_2$ to $C_{22}$ mono-olefinically unsaturated hydrocarbons. The present process is as applicable to the detergent range olefins, i.e. $C_8$ through $C_{22}$, as it is for the lower ranged olefins, $C_2$ through $C_7$. The invention is particularly suited for the lower range $C_2$ through $C_5$ olefins. Mixtures of olefins may be used. Specific examples include ethylene, propylene, butenes, pentenes, hexenes, cyclohexenes, heptenes, octenes, nonenes, dodecenes, pentadecenes and octadecenes. The present invention is particularly advantageous for hydrating propylene to isopropanol. Olefinically unsaturated compounds in admixture with non-olefinic material, e.g. alkanes are also suitably used, and a particularly useful feedstock for the present invention is a so-called BB or butane/butene stream which is mainly a mixture of isobutane with isomeric butenes. The products of the present invention are the alcohols corresponding to the hydration products of the olefinically unsaturated materials used.

The amounts of initial reactants used in the present invention may vary between wide limits. The moles of water initially present should be equal at least to the moles of unsaturated bonds in the olefin to be hydrated. The molar ratio of olefin to water will range from 1:1 to 1:50, preferably from 1:1 to

1:20, and most preferably from 1:1 to 1:10. The molar ratio of alpha-hydroxysulphonic acid to olefin will range from 1:100 to 5:1, preferably from 1:10 to 2:1.

The temperature at which the reaction is carried out will depend on several variables. One significant variable would be the specific reactant alcohol combination utilized. The higher molecular olefin and alcohols would require higher temperatures in order to maintain reasonable viscosities of the reacting and reactant materials. The particular alpha-hydroxysulphonic acid ultilized will also determine reaction temperatures and pressures. The reaction temperatures and pressures chosen should be such as to maintain a high proportion of alpha-hydroxysulphonic acid in the reaction mixture. Temperatures may range from —20°C to 200°C, preferably from 0°C to 100°C. Preferred pressures range from 0.5 to 5 kPa. The use of the lower molecular weight alpha-hydroxysulphonic acids will result in more optimal utilization of higher temperatures than would the use of the higher molecular weight acids. For example, alpha-hydroxymethanesulphonic acid begins to lose some of its efficiency above about 100°C (at one kPa) because of the equilibrium shift above this temperature to the component parts, whereas alpha-hydroxyethanesulphonic acid begins to lose some of its efficiency above about 40°C (at one kPa). Contact times may range from 0.01 to 100 h, preferably from 0.1 to 50 h. The contact of the olefin-containing hydrocarbon phase with the aqueous acid-containing phase involves intimate mixing of two immiscible phases by techniques that are well-known in the art.

Inert solvents may also be used in the process of the present invention. For example, non-polar organic solvents such as alkanes can be utilized to lower the viscosity of the reactant olefin component. Organic solvents which possess both hydrocarbon and water solubility may also be used. These latter materials comprise, for example, alcohols, ethers, ketones, acids and sulphones.

The reaction product is worked up by any convenient technique. For example, the hydration reaction product mixture can be subjected to vacuum to strip off the sulphur dioxide from the alpha-hydroxysulphonic acid, and then subsequently stripped by conventional techniques to remove unreacted olefinically unsaturated compounds, carbonyl compounds, and any other non-olefinic material present. The removed material may be recycled directly to the hydration reactor or the carbonyl compounds can be extracted and later combined with $SO_2$ and water to form the alpha-hydroxysulphonic acid which is then recycled to the hydration reactor. The stripped reaction product may then be distilled to produce the alcohol product.

The invention will be illustrated by reference to the following Example, which should not be construed as limiting the invention.

## Example

In a typical preparation of alpha-hydroxymethanesulphonic acid, 30 g of sulphur dioxide were condensed (dry ice/acetone bath) into a 200 ml Fisher-Porter pressure bottle. The reaction vessel was opened and slightly less than one equivalent of aqueous formaldehyde solution (37%w $H_2CO$) was added. The pressure bottle was sealed and the mixture warmed to room temperature. The mixture was stirred vigorously overnight and then vented ($SO_2$) to the atmosphere. Analysis of the resulting aqueous phase (potentiometric titration with $NaOH/H_2O$) found 5M alpha-hydroxymethanesulphonic acid and a trace of sulphur dioxide. The solution could be stored for up to about four weeks in the pressure bottle without loss in activity.

In a typical reaction 11 g (260 mmol) of propylene, 66 g (3600 mmol) of water and 270 mmol of acid catalyst were added to a 300 ml stainless steel autoclave. The reactor was heated to 85°C for the indicated time and initial pressure. The results determined by gaschromatography analysis are shown in Table I below. The sulphuric acid catalyst was noted to form sulphates and the p-toluenesulphonic acid catalyst was noted to have oxidized isopropyl alcohol to acetone. Neither of these by-products was noted with the alpha-hydroxysulphonic acid catalyst.

TABLE I

| Acid catalyst | Time (h) | Initial pressure (MPa) | Yield of isopropyl alcohol (%m) |
|---|---|---|---|
| Alpha-hydroxymethane-sulphonic acid | 2 | 2.22 | 11.7 |
| Alpha-hydroxymethane-sulphonic acid | 2 | 1.90 | 8.3 |
| Alpha-hydroxymethane-sulphonic acid | 14 | 2.28 | 9.8 |

Comparative experiments were carried out without the use of an alpha-hydroxysulphonic acid. The results are given in Table II below.

TABLE II

| Acid catalyst | Time (h) | Initial pressure (MPa) | Yield of isopropyl alcohol (%m) |
|---|---|---|---|
| $SO_2/H_2O$ control | 2 (80°C) | 3.45 | 0.2 |
| p-toluenesulphonic acid | 2 | 2.62 | 3.8 |
| $H_2SO_4$ | 2 | 2.52 | 4.6 |

Repeating the above experiment with propylene on 1-pentene and 1-dodecene utilizing alpha-hydroxymethanesulphonic acid as the catalyst gave similarly satisfactory yields of the corresponding alcohols, which were considerably higher than those obtained with the catalysts mentioned in Table II.

## Claims

1. A process for the preparation of alcohols, which comprises reacting an olefinically unsaturated hydrocarbon with water in the presence of a sulphonic group(s) — containing characterised in that the reaction is carried out in the presence of an alpha-hydroxysulphonic acid prepared by reacting a carbonyl compound of the general formula $R_1R_2CO$, wherein $R_1$ and $R_2$ are individually hydrogen or a substituted or unsubstituted hydrocarbyl group containing up to 7 carbon atoms, with sulphur dioxide and water.

2. A process as claimed in claim 1, wherein the initial molar ratio of olefinically unsaturated hydrocarbon to water ranges from 1:1 to 1:10.

3. A process as claimed in claim 1 or 2, wherein the initial molar ratio of acid to hydrocarbon ranges from 1:10 to 2:1.

4. A process as claimed in any one of claims 1—3, wherein the olefinically unsaturated hydrocarbon contains 2—22 carbon atoms.

5. A process as claimed in claim 4, wherein the olefinically unsaturated hydrocarbon contains 2—7 carbon atoms.

6. A process as claimed in claims 4 and 5, wherein the olefinically unsaturated hydrocarbon is propylene.

7. A process as claimed in any one of claims 1—6, wherein the acid is alpha-hydroxymethanesulphonic acid.

8. A process as claimed in any one of claims 1—7, wherein the process is carried out at a temperature ranging from 0 to 100°C.

## Revendications

1. Procédé de préparation d'alcools, dans lequel on fait réagir un hydrocarbure oléfiniqement insaturé avec de l'eau en présence d'un catalyseur contenant un ou plusieurs groupes sulfone, caractérisé en ce que la réaction est conduite en présence d'un acide alpha-hydroxysulfonique préparé en faisant réagir un composé carbonyle de formule générale $R_1R_2CO$, où $R_1$ et $R_2$ représentent individuellement un hydrogène ou un groupe hydrocarbyle substitué ou non substitué contenant jusqu'à 7 atomes de carbone, avec de l'anhydride sulfureux et de l'eau.

2. Procédé selon la revendication 1, où le rapport molaire initial de l'hydrocarbure oléfiniquement insaturé à l'eau va de 1:1 à 1:10.

3. Procédé selon l'une des revendications 1 ou 2, où le rapport molaire initial de l'acide à l'hydrocarbure va de 1:10 à 2:1.

4. Procédé selon l'une quelconque des revendications 1—3, où l'hydrocarbure oléfiniquement insaturé contient de 2 à 22 atomes de carbone.

5. Procédé selon la revendication 4, où l'hydrocarbure oléfiniquement insaturé contient de 2 à 7 atomes de carbone.

6. Procédé selon l'une des revendications 4 et 5, où l'hydrocarbure oléfiniquement insaturé est le propylène.

7. Procédé selon l'une quelconque des revendications 1—6, où l'acide est l'acide alpha-hydroxyméthanesulfonique.

8. Procédé selon l'une quelconque des revendications 1 à 7, où le procédé est conduit à une température allant de 0 à 100°C.

## Patentansprüche

1. Ein Verfahren zur Herstellung von Alkoholen, welches die Umsetzung eines olefinisch ungesättigten Kohlenwasserstoffs mit Wasser in Gegenwart eines (eine) sulfonische Gruppe(n) enthaltenden Katalysators umfaßt, dadurch gekennzeichnet, daß die Umsetzung in Gegenwart einer $\alpha$-Hydroxysulfonsäure, hergestellt durch die Umsetzung einer Carbonylverbindung der allgemeinen Formel $R_1R_2CO$, wobei $R_1$ und $R_2$ unabhängig voneinander Wasserstoff oder eine substituierte oder unsubstituierte Kohlenwasserstoffgruppe, enthaltend bis zu 7 Kohlenstoffatomen, sind, mit Schwefeldioxid und Wasser, durchgeführt wird.

2. Ein Verfahren wie in Anspruch 1 beansprucht, in welchem das molare Verhältnis von olefinisch ungesättigtem Kohlenwasserstoff zu Wasser im Bereich zwischen 1:1 und 1:10 liegt.

3. Ein Verfahren wie in Anspruch 1 oder 2 beansprucht, in welchem das anfängliche molare Verhältnis von Säure zu Kohlenwasserstoff im Bereich von 1:10 bis 2:1 liegt.

4. Ein Verfahren wie in irgendeinem der Ansprüche 1 bis 3 beansprucht, in welchem der olefinisch ungesättigte Kohlenwasserstoff 2 bis 22 Kohlenstoffatome enthält.

5. Ein Verfahren wie in Anspruch 4 beansprucht, in welchem der olefinisch ungesättigte Kohlenwasserstoff 2 bis 7 Kohlenstoffatome enthält.

6. Ein Verfahren wie in Anspruch 4 und 5 beansprucht, in welchem der olefinisch ungesättigte Kohlenwasserstoff Propylen ist.

7. Ein Verfahren wie in irgendeinem der Ansprüche 1 bis 6 beansprucht, in welchem die Säure $\alpha$-Hydroxymethanesulfonsäure ist.

8. Ein Verfahren wie in irgendeinem der Ansprüche 1 bis 7 beansprucht, in welchem das Verfahren bei einer Temperatur im Bereich von 0 bis 100°C durchgeführt wird.